Europäisches Patentamt

European Patent Office

Office européen des brevets

(11) Publication number: **0 165 807**
**B1**

# EUROPEAN PATENT SPECIFICATION

(45) Date of publication of patent specification: **30.08.89**

(51) Int. Cl.⁴: **A 61 F 13/16,** A 41 B 13/02,
A 61 F 13/00

(21) Application number: **85304374.3**

(22) Date of filing: **19.06.85**

(54) Sanitary napkin with gross foramina overlying a low density, resilient structure.

(30) Priority: **21.06.84 US 623274**

(43) Date of publication of application:
**27.12.85 Bulletin 85/52**

(45) Publication of the grant of the patent:
**30.08.89 Bulletin 89/35**

(84) Designated Contracting States:
**AT BE CH DE FR GB IT LI NL**

(56) References cited:
**CH-A- 608 698**
**DE-A-3 219 234**
**GB-A-1 160 625**
**US-A-3 371 667**
**US-A-3 759 262**
**US-A-3 897 784**
**US-A-4 397 644**

(73) Proprietor: **THE PROCTER & GAMBLE**
**COMPANY**
**One Procter & Gamble Plaza**
**Cincinnati Ohio 45202 (US)**

(72) Inventor: **Osborn III, Thomas Ward**
**400 Deanview Drive**
**Cincinnati Ohio 45224 (US)**

(74) Representative: **Gibson, Tony Nicholas et al**
**Procter & Gamble (NTC) Limited Whitley Road**
**Longbenton Newcastle upon Tyne NE12 9TS**
**(GB)**

Courier Press, Leamington Spa, England.

# Description

## Background of the invention
### Field of the invention

This invention relates to devices designed to be used in contact with the human body to absorb bodily discharges. Such devices include sanitary napkins, diapers, bandages and the like.

### Background art

Sanitary napkins, diapers, bandages, and the like intended for disposal after a single use are familiar items of modern commerce and are described in numerous articles and patents. For purposes of simplicity, the present invention will be described in terms of a sanitary napkin.

Mathews et al, in U.S. Patent 4,397,644 issued August 9, 1983, discuss a sanitary napkin comprising a cover (or topsheet), a resilient layer attached to the topsheet, an absorbent layer, and a fluid baffle (or backsheet). Since sanitary napkins are frequently intended to be supported adjacent the body through the agency of the user's undergarment (e.g. her panty), Matthews et al illustrate their sanitary napkin as being provided with garment fastening adhesive and an associated release liner. The cover is formed with pores having a particular size requirement designed to produce a "wet feeling surface" when the napkin is worn.

Fitzgerald in U.S. Patent No. 3,897,784 discloses a sanitary napkin formed of a three ply absorbent pad enclosed in a fluid-impermeable cover which is open on its body contacting surface. The uppermost ply of the absorbent pad is formed with large apertures to facilitate fluid transport into the interior plies of the pad.

Morse, in U.S. Patent 3,371,667 issued March 5, 1968 illustrates a conventional sanitary napkin having an outside foraminous cover (topsheet) and a highly absorbent, relatively dense core with a low density structure, such as a fibrous web of synthetic fibers, interposed between them. The foraminous cover is a woven or nonwoven fibrous material (usually cotton or rayon) having the normal random arrangement of apertures.

British Patent 1,160,625, published August 6, 1969, discusses a sanitary towel comprising an absorbent core member, an envelope (topsheet), and a wadding-like soft layer between them. The soft layer permits "liquid to pass directly therethrough to the absorbent core member without rendering possible, in any appreciable degree, absorbtion or lateral transportation of liquid in the soft fibrous layer." The envelope consists of a material "of a nature, such that liquid cannot be absorbed in or dispersed through the material of the envelope itself"; it is provided with perforations on the side of the towel intended to face to body.

## Summary of the invention

According to the present invention there is provided a device for absorbing bodily fluids comprising a fluid pervious cover forming a topsheet having an inner surface and an outer body-contacting surface having an impact zone, said fluid porous topsheet being provided with a large number of small apertures and on the part of said topsheet forming said body-contacting surface, a smaller number of larger apertures, an absorbent layer underlying said cover, a fluid impervious layer underlying said absorbent layer such that said absorbent layer is disposed between said fluid impervious layer and said body-contacting surface of said cover, adhesive garment attachment means secured to the outer surface of said device on the surface thereof opposed to said body-contacting surface and an intermediate layer disposed between said cover and said absorbent layer, wherein:

said topsheet is an apertured, formed, thermoplastic film;

the small apertures comprise fine foramina distributed over a major proportion of said body-contacting surface, each of said fine foramina having an equivalent hydraulic diameter, defined as 4A/P where A is the area and P the perimetric dimension of the foramen, of from 0.12 to 1.25 mm;

the large apertures comprise gross foramina disposed in at least said impact zone and each having an equivalent hydraulic diameter of from 1.25 to 9.5 mm; wherein

the ratio of the number of said gross foramina to said fine foramina is less than 0.25 and the ratio of the equivalent hydraulic diameters of said gross foramina to said fine foramina is greater than 2;

the intermediate layer comprises a resilient layer of uniformly low density that is capable of recovery to at least 80% of its original volume after it is compressed to 20% of its original volume and the compressing forces are removed, said resilient layer being absorbent, at least as hydrophilic as said apertured topsheet, and unattached thereto; and

a wicking layer disposed between said intermediate layer and said topsheet, adjacent the inner surface of said topsheet, and being non-extant in register with the gross foramina thereof, said wicking layer being more hydrophilic than said apertured topsheet.

Preferably the ratio of the equivalent hydraulic diameters of the gross end fine foramina is greater than 3.

The absorbent device of the invention comprises a topsheet which is generally pervious to liquids and which is provided with gross foramina (large apertures) at least in the zone normally subject to impact by the bodily discharge the device is designed to absorb. The gross foramina constitute a minority in number as compared to the fine foramina which tend to render the topsheet generally pervious to liquids. The topsheet, which is sometimes referred to as an "apertured topsheet", is a formed thermoplastic film. The apertured topsheet is hydrophobic and requires a wicking layer which is in contact with the apertured topsheet. The wicking layer is hydrophilic and has small absorbent capacity (at least in the impact zone discussed infra) as compared to other absorbent elements in the device. The wicking layer is nonextant in register with the gross foramina of the apertured topsheet, which is particularly important in those circumstances where the gross foramina are relatively large.

A resilient layer underlies the apertured topsheet. It is interposed between the apertured topsheet and the absorbent core described infra. While the resilient layer must exist in register with that region of the topsheet which is normally subject to impact by bodily discharges, it can extend over a major portion of the body-contacting surface of the absorbent device, and even along its sides; it preferably exists, however, only in the region subject to impact by bodily discharges. The resilient layer is formed primarily of a mass of hydrophobic, moisture insensitive, resilient fibers. Fiber dimensions are selected for both comfort and fluid transfer reasons and may take a range of values. In one embodiment the resilient fibers are bonded together to create a structure having even greater resilience. In another embodiment the resilient layer as a whole is actually hydrophilic, but is less hydrophilic than the absorbent core described infra.

In still another embodiment, the resilient layer comprises ancillary absorbent material such as the bibulous cellulosic fibers described in U.S. Patent 3,589,364 which issued to Dean and Ferguson on July 29, 1971 and the substantially insoluble acidic carboxymethylcellulose products described in U.S. Patent 3,678,031 which issued to Schoggen on July 18, 1972.

Other ancillary absorbent materials can include any of the so called super absorbent materials such as the cross-linked polyacrylates and cross-linked starch-acrylate polymers well known to those skilled in the art.

Fourth, there is an absorbent core which can be, in general, any absorbent material known to the art. Wood pulp fluff, comminuted sponge, supersorbent materials, and the like, alone and in combination, can be used. A fibrous core can be bonded or unbonded. The absorbent core must be more hydrophilic than the resilient layer.

Fifth, there is a liquid impermeable moisture barrier which can be any material well known to those skilled in the art. It can be completely impermeable, as polyethylene film, or it can be liquid impermeable but vapor permeable, as any of the various breathable backsheets known to the art.

Brief description of the drawings

Figure 1 is a plan view of a generalized sanitary napkin.

Figure 2 is a cross sectional view of the sanitary napkin of Figure 1 taken along line 2—2 of Figure 1. (In all of the cross sectional views, the thicknesses of certain materials have been exaggerated for clarity.)

Figure 3 is an enlarged fragmentary plan view of a portion of an absorbent device.

Figure 4 is a fragmentary cross sectional view of the absorbent device shown in Figure 3 taken along line 4—4 of Figure 3.

Detailed description of the invention

While this specification concludes with claims particularly pointing out and distinctly claiming that which is regarded as the invention, it is believed that the invention can be better understood through a purusal of the following detailed description of the invention in connection with study of the associated drawings.

As noted supra, for purposes of simplicity, the present invention will be discussed in terms of a sanitary napkin.

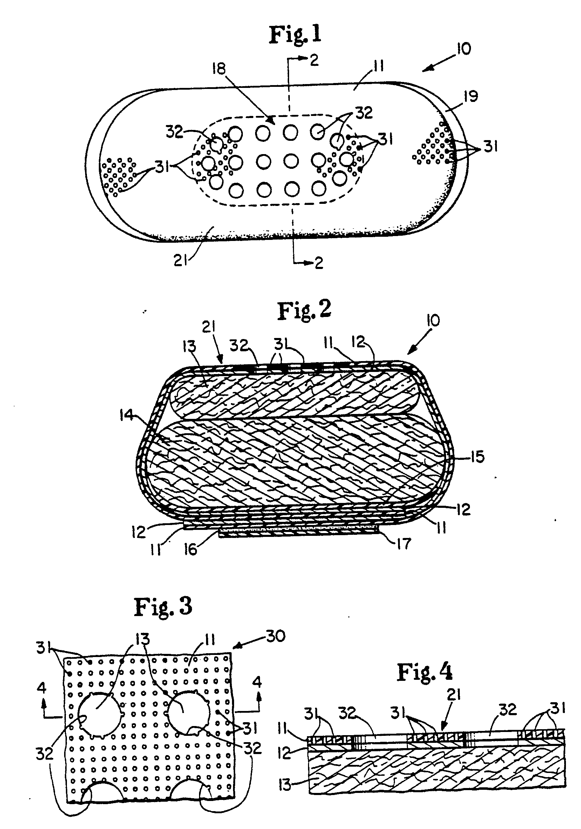

Figure 1 is a plan view of a generalized sanitary napkin 10. Figure 2 is a cross sectional view of sanitary napkin 10 taken along line 2—2 of Figure 1. (In this specification, all the reference numerals are used consistently among the various figures to denote equivalent and similar elements.)

As illustrated in Figure 1, sanitary napkin 10 is an elongate absorbent device intended to be maintained in a woman's crotch region to absorb vaginal discharges such as menses. The version of sanitary napkin 10 illustrated in Figure 1 has generally straight longitudinal edges and rounded ends. It is to be emphasized that this design is used for illustrative purposes only; any convenient design known to those skilled in the art can be used in the practice of the present invention. The sanitary napkin can, for example, have a generally hourglass shape wherein the longitudinal edges are curvilinear and the central portion is narrower in the lateral direction than are the end portions. Such a design is generally illustrated in U.S. Patent 4,324,246 issued to Mullane and Smith on April 13, 1982. The present invention can also be used with the compound sanitary napkin described by Des Marais in U.S. Patent 4,425,130 issued January 10, 1984. Further, the present invention can be used with the form of sanitary napkin having side panels described by McNair in U.S. Patent 4,285,343 issued August 25, 1981.

Likewise, the cross sectional view of sanitary napkin 10 shown in Figure 2 merely illustrates one typical configuration of a sanitary napkin employing the present invention. Skilled artisans can readily adapt the following teachings to other embodiments without departing from the scope and tenor of the present invention.

In this embodiment, panty fastening adhesive 16 is shown to be a single strip of adhesive which extends over a major portion of the longitudinal length of sanitary napkin 10 on the side of the device opposite body-contacting surface 21. Other configurations for the application of the panty fastening adhesive, such as multiple longitudinal stripes, can be used. Any of the pressure sensitive adhesives commonly used in the art, such as Century A-3051B adhesive manufactured by Century Adhesive Corporation and Instant Lok 34-2823 manufactured by National Starch Company, can be used. Panty fastening adhesive 16 is usually covered, prior to the time the user affixes the sanitary napkin to her undergarment, with release liner 17. Release liner 17 serves to keep panty fastening adhesive 16 from drying out and to keep it from sticking to extraneous surfaces prior to use. Any release liner commonly used for such purposes can be used with this invention. Nonlimiting examples of suitable release liners are BL30MG-A Silox E1-0 and BL30MG-A Silox 4P/0 manufactured by Akrosil Corporation.

Embodiments of sanitary napkins designed to be supported adjacent the user's body by various belts and the like can likewise benefit from the practice of the present invention.

Two of the necessary elements of the present invention comprise an apertured topsheet which is generally pervious to liquids and which is provided with gross foramina at least in the zone normally subject to impact by the bodily discharges the device is designed to absorb, and a resilient layer underlying the apertured topsheet.

The topsheet is the part of an absorbent device which contacts the user's body. Frequently it is also an overwrap for the device as illustrated in Figure 2. The topsheet must be soft, conformable, and nonirritating to the user's body.

Since the topsheet is the portion of the absorbent device which contacts the user's body, bodily discharges must pass through the topsheet into the body (or bulk) of the absorbent device. Bodily discharges such as menses frequently comprise fluids (of various viscosities), semi-fluid materials, and particulate materials. While many conventional prior art topsheets have been found to function adequately, many do not allow the efficient transport therethrough of semi-fluid and particulate materials. The present invention addresses this problem.

In Figures 1 and 2, apertured topsheet 11 is generally permeable to liquids and is provided with gross formina in the zone subject to impact by the bodily discharges the device is designed to absorb.

In the first place, the apertured topsheet must be generally permeable to fluids over most of that surface of the device which contacts the user's body; the permeability must be *separate, apart,* and *in addition to* that provided by the gross foramina discussed infra. Thus a material such as polyethylene film which is normally impermeable to fluids is not suitable.

Suitable apertured topsheets comprise formed thermoplastic films. As used herein, the term "formed thermoplastic film" or "formed film" refers to sheets which are a continuous layer of polymeric material which has been provided with embossments and apertures. Preferred formed films include those described with particularly in U.S. Patent 4,324,246 issued to Mullane and Smith on April 13, 1982; U.S. Patent 4,342,314 issued to Radel and Thompson on August 3, 1982; U.S. Patent 4,323,069 issued to Ahr and Smith on April 6, 1982; and U.S. Patent 3,929,135 issued to Thompson on December 30, 1975.

Permeability of the formed thermoplastic films is provided by the multiplicity of apertures (sometimes referred to as "fine foramina" and denoted by reference numeral 31 in Figure 2).

Apertured topsheet 11 must be provided with a multiplicity of gross foramina 32. While the entire surface 21 of apertured topsheet 11 which contacts the user's body can be provided with gross foramina, only that portion of apertured topsheet 11 comprising impact zone 18 (as delineated by the dotted line in Figure 1) must of necessity be so provided.

Impact zone 18 is illustrated in connection with sanitary napkin 10 as falling generally in the central region of body contacting surface 21. The exact location and size of impact zone 18 will vary according to the precise design and intended positioning of the sanitary napkin, diaper, bandage, or the like and can be readily ascertained by skilled artisans.

One important function of the topsheet of a sanitary napkin, disposable diaper, and the like is to present a dry surface in contact with the user's body even though bodily discharges have been absorbed by the device. In addition to relative surface hydrophobicity and the presence of the resilient layer as discussed infra, surface dryness is partially achieved by controlling the size of the fine foramina within specified limits. Exceeding these upper size limits normally destroys the ability of the topsheet to present a dry surface by allowing reverse flow of bodily discharges. It has been surprisingly discovered that the usual upper size limits of the fine foramina can be exceeded in a controlled fashion in an apertured topsheet provided the resilient layer discussed infra is also present in the device.

If the apertured topsheet achieves its general permeability through the presence of a major number of fine foramina having dimensions (such as Equivalent Hydraulic Diameters) in the range of from 0.005 to 0.050 inch (0.12 to 1.25 millimeters), then it may be provided with a minor number of gross foramina having dimensions of from 0.05 to 0.375 inch (1.25 to 9.53 millimeters). Preferably, the topsheet is provided with a major number of fine foramina having dimensions of from 0.010 to 0.035 inch (0.25 to 0.90 millimeter) and a minor portion of gross foramina having dimensions of from 0.050 to 0.375 inch (1.25 to 9.53 millimeters). The ratio of the dimensions of the gross foramina to the fine foramina should be greater than 2, preferably greater than 3. It is not necessary that all the fine foramina and all the gross foramina have equal sizes; a distribution of sizes is satisfactory. (Equivalent Hydraulic Diameter is defined as four times the quotient obtained when the area of a foramen is divided by its perimeter. In the case of circular foramina, the Equivalent Hydraulic Diameter is the diameter of the circle.)

The relationship between major and minor numbers depends upon the size distributions of the fine foramina and the gross foramina. Gross foramina should constitute no more than 20% of the total number of foramina in the apertured topsheet. Further, in general, if the fine foramina, without regard to the presence of the gross foramina, constitute from 15 to 52% open area (as measured by projection of the topsheet onto a plane), the gross foramina can comprise from 5 to 30% open area of the topsheet without regard to the fine foramina. Stated in an alternate fashion, if the topsheet is provided with fine foramina of Equivalent Hydraulic Diameter within the ranges mentioned above, and if the fine foramina, independently of the presence to the gross foramina, constitute from 15 to 52% open area of the topsheet then the number of gross foramina per unit area (the gross foramina having dimensions falling within the ranges mentioned above) can be selected so that the gross foramina provide from 5 to 30% open area independently of the open area provided by the fine foramina so long as the ratio of numbers of gross foramina to fine foramina is less than about 1 to 4 (0.25).

The exact shape of the gross and fine foramina is not critical; circular foramina are satisfactory.

The state of the art respecting wetting of materials allows for the definition of hydrophobicity and hydrophilicity in terms of contact angles and the surface tensions of the fluids and solids involved. This is discussed in detail in the American Chemical Society Publication *Contact Angle, Wettability, and Adhesion* edited by Robert F. Gould and copyrighted in 1964.

A surface is said to be wetted by fluid either when the contact angle between the fluid and the surface is less than 90° or when the fluid will tend to spread spontaneously across the surface; both conditions normally coexist. In a similar vein, a surface is said to be not wetted by a fluid when the contact angle between the fluid and the surface is greater than 90° and when the fluid does not tend to spread spontaneously across the surface.

Since menses and other bodily fluids are primarily aqueous solutions, materials which are wetted by these fluids can broadly be described as hydrophilic, and surfaces which are not wetted by these fluids can be broadly described as hydrophobic. Unless otherwise indicated, as used in this specification the term "hydrophobic" describes materials and surfaces which are not wetted by the fluid in question while the term "hydrophilic" describes materials and surfaces which are wetted by the fluid in question.

Vaginal discharges normally have a surface tension of from 35 to 60 dynes per centimeter. They will have contact angles of greater than 90° and will not tend to spread spontaneously across a solid surface which has a critical surface tension or energy value lower than the fluid surface tension. As a first approximation, any solid which is not wetted by water (i.e. which is literally hydrophobic in the precise, limited meaning of the word) is also usually not wetted by vaginal discharges.

In absorbent devices of the present invention, apertured topsheets 11 is hydrophobic. The absorbent device is therefore provided with a wicking layer 12 affixed to the inner surface of apertured topsheet 11. The "inner surface" of apertured topsheet 11 is that surface of apertured topsheet 11 which is opposite body-contacting surface 21. In the normal construction, wicking layer 12 is interposed between apertured topsheet 11 and resilient layer 13 described infra and, in general, the balance of the absorbent device.

The purpose of the wicking layer is to induce fluids to flow through apertured topsheet 11 and away from body-contacting surface 21. Wicking layer 12 should be more hydrophilic (or, conversely, less hydrophobic) than apertured topsheet 11.

Wicking layer 12 can be provided by any convenient means. For example, it can comprise a tissue paper sheet made according to the teachings of Sanford and Sisson in U.S. Patent 3,301,746 issued January 31, 1967 or according the to teachings of Morgan and Rich in U.S. Patent 3,994,771 issued November 30, 1976.

An especially suitable tissue is sold under the registered trademark BOUNTY by Procter & Gamble of Cincinnati, Ohio.

Preferably, wicking layer 12 comprises a multiplicity of individual fibers which are evenly dispersed over and affixed to the inner surface of apertured topsheet 11. These fibers can be of any suitable material so long as they are more hydrophilic than apertured topsheet 11. For example, fibers of polyester, rayon, nylon, and cotton can be used with good results. Wood pulp fibers are, however, preferred. Typically, at least 1.5 grams and preferably at least 3.1 grams, of fibers are uniformly distributed and adhesively affixed to the inner surface of apertured topsheet 11. An acrylic adhesive, such as that marketed under the trademark Roplex HA-8 by Rohm & Haas Company of Philidelphia can be used.

Wicking layer 12 is continuous in those regions of the inner surface of apertured topsheet 11 in register with fine foramina 31. Preferably, wicking layer 12 does not exist in those regions in register with gross foramina 32; the degree of preferance increases as the absolute size of gross foramina 32 increases.

Figure 3 is an enlarged fragmentary plan view of a portion of an absorbent device 30 embodying the teachings of the present invention. A small fraction of the fine foramina 31 with which apertured topsheet 11 is provided are illustrated as circular apertures. Gross foramina 32 are also illustrated as circular apertures.

Figure 4 is an enlarged fragmentary cross sectional view taken along line 4—4 of Figure 3. Figures 3 and 4 illustrate the preferred embodiment wherein wicking layer 12 exists in register with a significant number of fine foramina 31, but does not exist in register with gross foramina 32. Thus, bodily discharges have an essentially uninterupted passage from body-contacting surface 21 of apertured topsheet 12 to resilient element 13.

In Figure 2, wicking layer 12 is shown to be coextensive with apertured topsheet 11 throughout the structure of sanitary napkin 10. This form of construction results from the convenience of providing apertured topsheet 11 and wicking layer 12 affixed one to another as a unit during the construction of sanitary napkin 10. Wicking layer 12 need be coextensive with apertured topsheet 11 only in those regions of apertured topsheet 11 which contact the user's body and which receive bodily discharges.

Another necessary element of the absorbant device of this invention is a resilient layer as indicated by reference numeral 13 in Figure 2.

The resilient layer serves several functions in the absorbent device. For example, it tends to isolate the apertured topsheet from bodily discharges which have already passed through the apertured topsheet into the absorbent device. Because of its physical characteristics, as described infra, the resilient layer enhances the comfort perceived by the user of the absorbent device. It also serves as a reservoir for bodily discharges although this function is of lesser importance because of the presence of the absorbent core described infra.

Resilient layer 13 must possess certain physical characteristics. It must be resilient. That is to say, it must, without the application of external forces, return to essentially its original size and shape after deforming forces (such as those applied by the body of the user) are removed. For the purposes of the present invention, the resilient layer possesses such resilience that it will recover at least 80% of its original volume after it is compressed to 20% of its original volume and the compressing forces are removed. Its resilience must be essentially unaffected by the presence of moisture such as the moisture in bodily discharges; that is to say, the resilient layer must be essentially moisture insensitive.

The resilient layer must be compressible and conformable. In other terms, it must possess such physical properties of compressibility and conformability that, when it is in contact with the user's body and subjected to forces applied by the user's body and clothing, it will conform to the user's body without causing a feeling of discomfort to the user.

Finally, the resilient layer must be at least as hydrophilic as the apertured topsheet.

Preferably, resilient layer 13 comprises a mass or batt of fibers. Synthetic fibers useful in the present invention include those made of cellulose acetate, polyvinyl chloride, polyvinylidene chloride, acrylic resins, polyvinyl acetates, non-soluble polyvinyl alcohols, polyethylenes, polypropylenes, polyamids, and preferably, polyesters. Preferred polyester fibers have a density of from 0.44 to 6.6 Tex and a length of from 2 to 8 centimeters. The resilience of the resilient element can frequently be enhanced if the fibers are bonded one to another at a significant number of their points of contact by adhesive or thermal bonding as well known to those skilled in the art.

In those circumstances where the inherent hydrophobicity of the resilient layer must be alleviated (as when it is constructed from a hydrophobic material), a degree of hydrophilicity can be imparted to the resilient layer by treating it with surfactant, such as a nonionic or an anionic surfactant. It can be sprayed with a surfactant or dipped into a solution of a surfactant. Suitable surfactants include nonionic surfactants such as Brij 76 manufactured by ICI Americans Inc. of Wilmington, Deleware and the various materials sold under the Pegosperse trademark by Glyco Chemicals, Inc. of Grenwich, Connecticut. Anionic surfactants can be used, but are not preferred.

Since bodily discharges will, in general, pass through, or at least be contained by, the resilient element, it must be of relatively low density so that it will have sufficient void volume to accomplish its intended purpose. Low density also helps to ensure that the resilient element deforms under the influence of the user's body thereby exhibiting comfort attributes. Preferably, the resilient element has a density of from 0.01 to 0.5 gram per cubic centimeter.

The second element of the absorbent device of the present invention is absorbent core 14.

Absorbent core 14 is, as its name implies, intended to absorb the bulk of the bodily fluids discharged by the user. Thus, the absorbent core must be constructed so as to readily absorb such fluids.

In general, the absorbent core comprises a material which is hydrophilic. This material can achieve hydrophilicity by any convenient means. For example, the material itself can be intrinsically hydrophilic. Alternatively, the material can be provided with a finish which renders its surfaces hydrophilic. Another method of rendering the material hydrophilic is to treat it with a surfactant, such as one of the nonionic surfactants mentioned above or an anionic surfactant, as by spraying the material with a surfactant or dipping the material into the surfactant.

A preferred material of construction for the absorbent core comprises airlaid wood pulp fibers. These fibers, sometimes known as comminution grade wood pulp fibers are fibers which have been liberated from the wood by any convenient means and dried, likewise by any convenient means. The resulting sheets of fibers are then comminuted to produce essentially intact individual fibers in a gaseous stream from which the fibers are then formed into an absorbent batt. Such absorbent batts are well known to those skilled in the sanitary napkin, diaper, and bandage art. Absorbent cores formed from such batts, as well as those described infra, can be of greater thicknesses in the central regions of the absorbent device than at the ends or edges or both the ends and the edges of the device.

Alternatively, but less preferably, the absorbent core can be made from an absorbent foam material such as cellulose foam or polyurethane foam. The latter, for example, can be made according to the teachings of U.S. Patent 4,067,832 which issued to DesMarais on January 10, 1978. Another particularly useful foam material is the polyester foam made according to the teachings of U.S. Patent 4,110,276 which issued to DesMarais on August 29, 1978.

Cellulose wadding as sometimes used in disposable diapers can also be used to construct the absorbent core. The absorbent core can be constructed from layers of tissue paper such as that mentioned supra.

Another material from which the absorbent core can be constructed comprises an unbonded array of nonabsorbent hydrophilic, resilient, moisture insensitive fibers as described in U.S. Patent 44-75911 which issued to Gellert on October 9, 1984.

Preferably, the absorbent core is made from airlaid wood fibers and has a density of from 0.003 to 0.09 gram per cubic centimeter.

Optionally, the absorbent core can contain ancillary absorbent material which tends to increase its absorbent capacity. Suitable ancillary absorbent materials include those discussed supra. The ancillary absorbent materials can be in fibrous or particulate form, and they can be either uniformly distributed throughout the bulk of the absorbent core or they can be concentrated in localized regions thereof.

Another element of the present invention is a moisture barrier as illustrated by reference numeral 15 in Figure 2. The moisture barrier, which is sometimes called a backsheet, serves to prevent bodily discharges contained within the absorbent device from passing completely through the absorbent device and soiling the user's body and clothing. Any of the materials commonly used for moisture barriers in the sanitary napkin and disposable diaper art can be used in the practice of this invention. Suitable materials include the well known fluid impermeable polyethylene films. The moisture barrier can also comprise the liquid impermeable, but vapor permeable materials sometimes known as breathable backsheets. Examples of such breathable backsheets are shown in U.S. Patent 3,881,489 issued to Hartwell on May 6, 1975, and U.S. Patent 3,989,867 issued to Sisson on November 2, 1976. Obenour, in U.S. Patent 4,341,216 issued July 27, 1982, describes a two element breathable backsheet useful in disposable diapers which can be used in the practice of the present invention.

Ferguson and Landrigan U.S. Patent 4341217 describes a barrierless disposable absorbent article encased in a homogeneous outerwrap. The techniques described in that patent can be used to provide an equivalent of the moisture barrier just discussed.

In addition to a moisture barrier comprising a separate element, an equivalent result can be obtained by treating the undersurface of the absorbent core with a material, such as silicone or a wax, which will render a portion of the absorbent core liquid impermeable.

In Figure 2, moisture barrier 15 is illustrated as being completely encased within apertured topsheet 11 which then functions as an overwrap for sanitary napkin 10 and is sealed along end seals 19 by any convenient means such as adhesive sealing. This form of construction is illustrative only; those skilled in the art can readily supply alternate design details.

The invention has been described in terms of a sanitary napkin. It has been noted several times previously that these basic teachings can be readily applied by skilled artisans to other absorbent devices such as disposable diapers. Diaper designs suitable for use with the present invention include those described in U.S. Patent Re. 26,151 which was issued to Duncan and Baker on January 31, 1967 and U.S. Patent 3,860,003 which was issued to Buell on January 14, 1975.

By way of illustration and not by way of limitation, the following example is presented.

Example 1

A sanitary napkin having the planform illustrated in Figure 1 and the cross-sectional configuration illustrated in Figure 2 is constructed. The apertured topsheet is formed thermoplastic film having round gross foramina 6.35 mm in diameter and fine foramina 0.76 mm in diameter. There are 60 fine foramina for every gross foramen. The wicking layer comprises a single ply of BOUNTY tissue and is attached to the apertured topsheet with Roplex HA-8 adhesive. The wicking layer is non-extant in register with the gross foramina. The resilient element is a latex-bonded polyester nonwoven manufactured by Stearns & Foster Company of Cincinnati, Ohio. The fiber type is 1.67 Tex Kodell Type 430 made by E. I. Dupont de Nemours & Company; the latex binder is UCAR 879 made by Union Carbide Co. and is applied at 15% by weight of the fiber. The resilient layer is 11.4 mm thick and has a density of 0.01 gram per cubic cm; it is treated with Pegosperse 200 ML. The absorbent core consists of 3 grams airlaid comminution pulp as made by The Buckeye Cellulose Corporation of Memphis, Tenn. The moisture barrier is polyethylene film. The panty fastening adhesive is Century A-3051 B adhesive and is covered by BL30MG-A Silox E1-0 release liner.

The sanitary napkin, which is 21.6 cm long by 6.4 cm wide, performs effectively and is comfortable to wear.

**Claims**

1. A device (10) for absorbing bodily fluids comprising a fluid pervious cover forming a topsheet (11) having an inner surface and an outer body-contacting surface (21) having an impact zone (18), said fluid porous topsheet being provided with a large number of small apertures (31) and on the part of said topsheet forming said body-contacting surface, a smaller number of larger apertures (32), an absorbent layer (14) underlying said cover, a fluid impervious layer (15) underlying said absorbent layer such that said absorbent layer is disposed between said fluid impervious layer and said body-contacting surface of said cover, adhesive garment attachment means (16, 17) secured to the outer surface of said device on the surface thereof opposed to said body-contacting surface and an intermediate layer (13) disposed between said cover and said absorbent layer, characterised in that:

said topsheet (11) is an apertured, formed, thermoplastic film;

the small apertures (31) comprise fine foramina distributed over a major proportion of said body contacting surface, each of said fine foramina having an equivalent hydraulic diameter, defined as 4A/P where A is the area and P the perimetric dimension of the foramen, of from 0.12 to 1.25 mm;

the large apertures (32) comprise gross foramina disposed in at least said impact zone (18) and each having an equivalent hydraulic diameter of from 1.25 to 9.5 mm; wherein

the ratio of the number of said gross foramina to said fine foramina is less than 0.25 and the ratio of the equivalent hydraulic diameters of said gross foramina to said fine foramina is greater than 2;

the intermediate layer (13) comprises a resilient layer of uniformly low density that is capable of recovery to at least 80% of its original volume after it is compressed to 20% of its original volume and the comprising forces are removed, said resilient layer being absorbent, at least as hydrophilic as said apertured topsheet, and unattached thereto; and

a wicking layer (12) disposed between said intermediate layer (13) and said topsheet (11), adjacent the inner surface of said topsheet, and being non extant in register with the gross foramina (32) thereof, said wicking layer (12) being more hydrophilic than said apertured topsheet (11).

2. A device according to claim 1 wherein the ratio of the equivalent hydraulic diameters of the gross (32) and fine (31) foramina is greater than 3.

3. A device according to either one of claims 1 and 2 wherein the equivalent hydraulic diameters of said fine foramina (31) are in the range from 0.25 to 0.90 mm.

**Patentansprüche**

1. Einrichtung (10) zum Absorbieren von Körperflüssigkeiten, umfassend eine eine Abdecklage (11) bildende, flüssigkeitsdurchlässige Abdeckung, wobei die Abdecklage eine Innenseite und eine mit dem Körper in Berührung kommende Außenseite (21) mit einer Auftreffzone (18) aufweist, une wobei diese flüssigkeitsdurchlässige Abdecklage mit einer großen Anzahl von kleinen Öffnungen (31) ausgestattet ist, und auf demjenigen Teil der Abdecklage, der die mit dem Körper in Berührung kommende Oberfläche bildet, mit einer kleineren Anzahl von größeren Öffnungen (32) ausgestattet ist; eine absorbierende Schicht 14, welche unterhalb der Abdeckung angeordnet ist; eine flüssigkeitsundurchlässige Schicht (15), welche unterhalb dieser absorbierenden Schicht angeordnet ist, so daß diese absorbierende Schicht zwischen der flüssigkeits-

unduchlässigen Schicht und der mit dem Körper in Berührung kommenden Oberfläche der Abdeckkung angeordnet ist; Befestigungsmittel (16, 17) zum Abkleben der Einrichtung an einem Wäschestück, welche Befestigungsmittel an der Außenseite der Einrichtung, und zwar auf derjenigen Oberfläche derselben, welche zu der mit dem Körper in Berührung kommenden Oberfläche entgegengesetzt angeordnet ist, befestigt sind; und eine Zwischenschicht (13), welche zwischen der Abdeckung und der absorbierenden Schicht angeordnet ist; dadurch gekennzeichnet, daß:

die Abdecklage (11) ein gelochter, geformter, thermoplastischer Film ist;

die kleinen Öffnungen (31) Feinöffnungen umfassen, welche über einem Hauptanteil der mit dem Körper in Berührung kommenden Oberfläche verteilt sind, wobei jede dieser Feinöffnungen einen äquivalenten hydraulischen Durchmesser von 0,12 bis 1,25 mm aufweist, und wobei der äquivalente hydraulische Durchmesser als 4A/P definiert ist, worin A die Fläche und P die Umfangserstreckung der Öffnung ist;

die großen Öffnungen (32) Groböffnungen umfassen, welche wenigstens in der Auftreffzone (18) angeordnet sind, wobei jede dieser Groböffnungen einen äquivalenten hydraulischen Durchmesser von 1,25 bis 9,5 mm aufweist; und wobei das Verhältnis von der Anzahl der Groböffnungen zu der Anzahl der Feinöffnungen weniger als 0,25 beträgt, und wobei das Verhältnis der äquivalenten hydraulischen Durchmesser der Groböffnungen zu denjenigen der Feinöffnungen größer als 2 ist;

die Zwischenschicht (13) eine elastische Schicht von gleichförmig niedriger Dichte umfaßt, welche elastische Schicht befähigt ist, auf wenigstens 80% ihres ursprünglichen Volumens zurückzufedern, nachdem sie auf 20% ihres ursprünglichen Volumens komprimiert worden ist und die komprimierenden Kräfte entfernt worden sind, wobei diese elastische Schicht absorbierend, wenigstens so hydrophil wie die gelochte Abdecklage, und an der letztgenannten nicht befestigt ist; und

eine dochtartig aufsaugende Schicht (12) zwischen der Zwischenschicht (13) und der Abdecklage (11), neben der Innenseite der Abdecklage, angeordnet, nicht vorstehend und deckungsgleich mit den Groböffnungen (32) der Abdecklage ist, wobei diese dochtartig aufsaugende Schicht (12) stärker hydrophil als die gelochte Abdecklage (11) ist.

2. Einrichtung nach Anspruch 1, worin das Verhältnis der äquivalenten hydraulischen Durchmesser der Groböffnungen (32) zu denjenigen der Feinöffnungen (31) größer als 3 ist.

3. Einrichtung nach Anspruch 1 oder 2, worin die äquivalenten hydraulischen Durchmesser der Feinöffnungen (31) in den Bereich von 0,25 bis 0,90 mm fallen.

**Revendications**

1. Dispositif (10) pour absorber les fluides corporels, comprenant une couverture perméable aux fluides formant une feuille de dessus (11) ayant une surface interne et une surface externe (21) en contact avec le corps comportant une zone d'impact (18), ladite feuille de dessus poreuse aux fluides étant munie d'un grand nombre de petites ouvertures (31) et, sur la partie de ladite feuille de dessus formant la surface en contact avec le corps, d'un petit nombre d'ouvertures plus grosses (32), une couche absorbante (14) située au-dessous de la couverture, une couche (15) imperméable aux fluides située au-dessous de la couche absorbante de telle manière que la couche absorbante soit disposée entre la couche imperméable aux fluides et la surface en contact avec le corps de la couverture, des moyens adhésifs (16, 17) de fixation aux vêtements attachés à la surface externe du dispositif sur sa surface opposée à la surface en contact avec le corps et une couche intermédiaire (13) disposée entre la couverture et la couche absorbante, caractérisé en ce que:

la feuille de dessus (11) est un film thermoplastique formé ajouré;

les petites ouvertures (31) comprennent des trous fins répartis sur une majeure partie de la surface en contact avec le corps, chacun de ces trous fins ayant un diamètre hydraulique équivalent, défini comme étant 4A/P, où A est la surface et P le périmètre du trou, de 0,12 à 1,25 mm;

les grosses ouvertures (32) comprennent de gros trous disposés au moins dans la zone d'impact (18) et ayant chacun un diamètre hydraulique équivalent de 1,25 à 9,5 mm;

le rapport du nombre des gros trous au nombre des trous fins étant inférieur à 0,25 et le rapport des diamètres hydrauliques équivalents des gros trous à ceux des trous fins étant supérieur à 2;

la couche intermédiaire (13) comprend une couche élastique de densité uniformément faible qui est capable de recouvrer au moins 80% de son volume initial après avoir été comprimée à 20% de son volume initial et après la suppression des forces de compression, cette couche élastique étant absorbante, au moins aussi hydrophile que la feuille de dessus ajourée, et non attachée à celle-ci; et

une couche à effet de mèche (12) disposée entre la couche intermédiaire (13) et la feuille de dessus (11), adjacente à la surface interne de la feuille de dessus et n'étant pas en coïncidence avec les grosses ouvertures (32) de celle-ci, cette couche à effet de mèche (12) étant plus hydrophile que la feuille de dessus ajourée (11).

2. Dispositif selon la revendication 1, dans lequel le rapport des diamètres hydrauliques équivalents des gros trous (32) et des trous fins (31) est supérieur à 3.

3. Dispositif selon l'une quelconque des revendications 1 et 2, dans lequel les diamètres hydrauliques équivalents des trous fins (31) sont dans l'intervalle de 0,25 à 0,90 mm.

**Fig.1**

**Fig.2**

**Fig.3**

**Fig.4**